(19) Europäisches Patentamt
European Patent Office
Office européen des brevets

(11) Publication number: **0 430 709 A2**

(12) **EUROPEAN PATENT APPLICATION**

(21) Application number: **90313061.5**

(22) Date of filing: **30.11.90**

(51) Int. Cl.[5]: **C07D 409/06**, C07D 409/14, A61K 31/41, C07D 333/52

(30) Priority: **01.12.89 GB 8927277**
**18.05.90 GB 9011186**

(43) Date of publication of application:
**05.06.91 Bulletin 91/23**

(84) Designated Contracting States:
**AT BE CH DE DK ES FR GB GR IT LI LU NL SE**

(71) Applicant: **GLAXO GROUP LIMITED**
**Clarges House 6-12 Clarges Street**
**London W1Y 8DH (GB)**

(72) Inventor: **Ross, Barry Clive**
**Glaxo Group Research Limited, Park Road**
**Ware, Hertfordshire SG12 0DG (GB)**
Inventor: **Middlemiss, David**
**Glaxo Group Research Limited, Park Road**
**Ware, Hertfordshire SG12 0DG (GB)**
Inventor: **Scopes, David Ian Carter**
**Glaxo Group Research Limited, Park Road**
**Ware, Hertfordshire SG12 0DG (GB)**
Inventor: **Jack, Torquil Iain Maclean**
**Glaxo Group Research Limited, Park Road**
**Ware, Hertfordshire SG12 0DG (GB)**
Inventor: **Cardwell, Kevin Stuart**
**Glaxo Group Research Limited, Park Road**
**Ware, Hertfordshire SG12 0DG (GB)**
Inventor: **Dowle, Michael Dennis**
**Glaxo Group Research Limited, Park Road**
**Ware, Hertfordshire SG12 0DG (GB)**

(74) Representative: **James, Stephen Richard et al**
**Glaxo Holdings plc 63 Graham Street**
**London N1 8JZ (GB)**

(54) **Benzthiophen derivatives.**

(57) The invention provides compounds of the general formula (I)

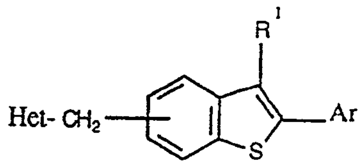

(I)

or a physiologically acceptable salt, solvate or non-toxic metabolically labile ester thereof wherein
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy, —CHO, —$CO_2H$ or —$COR^2$;
Ar represents the group

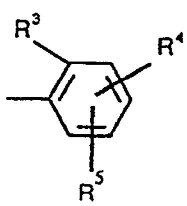

;

$R^2$ represents a group selected from $C_{1-6}$alkyl, $C_{2-6}$alkenyl, $C_{1-6}$alkoxy or the group —$NR^{10}R^{11}$;
$R^3$ represents a group selected from —$CO_2H$, —$NHSO_2CF_3$ or a C-linked tetrazolyl group;
$R^4$ and $R^5$, which may be the same or different, each independently represent a hydrogen atom or a halogen atom or a $C_{1-6}$alkyl group;
Het represents an N-linked imidazolyl group optionally substituted at the 2-position by a $C_{1-6}$alkyl, $C_{2-6}$alkenyl or a $C_{1-6}$alkylthio group, the imidazolyl group optionally being substituted at the 4- and 5-positions by one or two further substituents selected from a halogen atom or group selected from cyano, nitro, $C_{1-6}$alkyl, $C_{2-6}$alkenyl, fluoro$C_{1-6}$alkyl, —$(CH_2)_mR^6$, —$(CH_2)_nCOR^7$ or —$(CH_2)_pNR^8COR^9$;
$R^6$ represents a hydroxy or $C_{1-6}$alkoxy group;



Jouve, 18, rue Saint-Denis, 75001 PARIS

$R^7$ represents a hydrogen atom or a group selected from hydroxy, $C_{1-6}$alkyl, $C_{1-6}$alkoxy, phenyl, phenoxy or the group $-NR^{10}R^{11}$ ;
$R^8$ represents a hydrogen atom or a $C_{1-6}$alkyl group ;
$R^9$ represents a hydrogen atom or a group selected from $C_{1-6}$alkyl,$C_{1-6}$alkoxy, phenyl, phenoxy or the group $-NR^{10}R^{11}$ ;
$R^{10}$ and $R^{11}$, which may be the same or different, each independently represent a hydrogen atom or a $C_{1-4}$alkyl group or $-NR^{10}R^{11}$ forms a saturated heterocyclic ring which has 5 or 6 ring members and may optionally contain in the ring one oxygen atom ;
m represents an integer from 1 to 4 ;
n represents an integer from 0 to 4 ; and
p represents an integer from 1 to 4.
The compounds may be used in the treatment or prophylaxis of hypertension and diseases associated with cognitive disorders.

## BENZTHIOPHEN DERIVATIVES

This invention relates to benzthiophen derivatives, processes for their preparation and pharmaceutical compositions containing them. According to a first aspect of the invention we provide a compound of the general formula (I) :

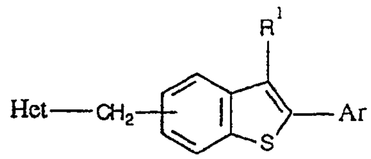

(I)

or a physiologically acceptable salt, solvate (e.g. hydrate) or metabolically labile ester thereof in which
$R^1$ represents a hydrogen atom or a halogen atom or a group selected from $C_{1-6}$ alkyl, $C_{2-6}$ alkenyl, fluoro$C_{1-6}$alkyl, $C_{1-6}$alkoxy, —CHO, —$CO_2H$ or —$COR^2$ ;
Ar represents the group

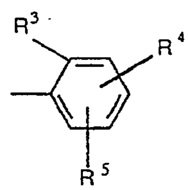

$R^2$ represents a group selected from $C_{1-6}$ alkyl, $C_{2-6}$ alkenyl, $C_{1-6}$ alkoxy or the group —$NR^{10}R^{11}$ ;
$R^3$ represents a group selected from —$CO_2H$, —$NHSO_2CF_3$ or a C-linked tetrazolyl group ;
$R^4$ and $R^5$ which may be the same or different each independently represent a hydrogen atom or a halogen atom or a $C_{1-6}$ alkyl group ;
Het represents an N-linked imidazolyl group optionally substituted at the 2-position by a $C_{1-6}$alkyl, $C_{2-6}$alkenyl or a $C_{1-6}$alkylthio group, the imidazolyl group optionally being substituted at the 4-and 5-positions by one or two further substituents selected from a halogen atom or a group selected from cyano, nitro, $C_{1-6}$alkyl, $C_{2-6}$alkenyl, fluoro$C_{1-6}$alkyl, —$(CH_2)_mR^6$, —$(CH_2)_nCOR^7$, or —$(CH_2)_pNR^8COR^9$ ;
$R^6$ represents a hydroxy or $C_{1-6}$alkoxy group ;
$R^7$ represents a hydrogen atom or a group selected from hydroxy, $C_{1-6}$alkyl, $C_{1-6}$alkoxy, phenyl, phenoxy or the group —$NR^{10}R^{11}$,
$R^8$ represents a hydrogen atom or a $C_{1-6}$alkyl group ;
$R^9$ represents a hydrogen atom or a group selected from $C_{1-6}$alkyl, $C_{1-6}$alkoxy, phenyl, phenoxy or the group —$NR^{10}R^{11}$ ;
$R^{10}$ and $R^{11}$, which may be the same or different, each independently represent a hydrogen atom or a $C_{1-4}$ alkyl group or —$NR^{10}R^{11}$ forms a saturated heterocyclic ring which has 5 or 6 ring members and may optionally contain in the ring one oxygen atom ;
m represents an integer from 1 to 4, preferably 1 or 2, especially 1 ;
n represents an integer from 0 to 4, preferably 0, 1 or 2, especially 0 or 1 ; and
p represents an integer from 1 to 4, preferably 1 or 2..

Where optical isomers may exist formula (I) is intended to cover all enantiomers, diastereoisomers and mixtures thereof including racemates. Compounds containing one or two double bonds may exist in the cis or trans configuration.

The invention also includes within its scope the solvates, especially the hydrates of compounds of general formula (I).

Within the above definition the term 'alkyl' or 'alkoxy' as a group or part of a group means that the group is straight or branched. The term 'alkenyl' as a group or part of a group means that the group is straight or branched and contains at least one carbon-carbon double bond.

The term 'halogen' means a fluorine, chlorine, bromine or iodine atom.

The term 'fluoro$C_{1-6}$alkyl' means a $C_{1-6}$alkyl group in which one or more hydrogen atoms have been replaced by a fluorine atom, for example, $-CH_2CF_3$. Particularly preferred are 'perfluoro$C_{1-3}$alkyl' groups meaning a fully fluorinated $C_{1-3}$alkyl group, i.e. trifluoromethyl, pentafluoroethyl, heptafluoropropyl or heptafluoroisopropyl.

Within the above definition when $-NR^{10}R^{11}$ represents a saturated heterocyclic ring, this contains 5 or 6 ring members, one of which may be an oxygen atom. Suitable heterocyclic groups are a pyrrolidino, piperidino or morpholino group.

A preferred class of compounds of general formula (I) is that wherein the group Het is substituted at the 2-position by a hydrogen atom or a $C_{1-5}$alkyl, especially a $C_{3-5}$ alkyl group or a $C_{3-5}$alkenyl group. Particularly preferred are those compounds wherein the 2-position substituent is an ethyl, n-propyl or n-butyl group, especially an n-butyl group. Conveniently, the $C_{3-5}$alkyl group may be a but-1-enyl group.

Another preferred class of compounds of general formula (I) is that wherein the group Het is optionally substituted by one or two further substituents selected from a halogen atom or a group selected from $C_{1-6}$alkyl, $-(CH_2)_mR^6$ or $-(CH_2)_nCOR^7$. In particular, $R^6$ represents a hydroxy or $C_{1-6}$alkoxy group, and preferably a hydroxy, methoxy, ethoxy, propoxy or butoxy group, and especially a hydroxy or methoxy group. $R^7$, in particular, represents a hydrogen atom or a hydroxy, $C_{1-6}$ alkoxy or $-NR^{10}R^{11}$ group (especially wherein $R^{10}$ and $R^{11}$ each independently represent a hydrogen atom or a $C_{1-4}$alkyl group), and preferably a hydrogen atom or a hydroxy, methoxy, ethoxy, propoxy or butoxy group, and especially a hydrogen atom or a hydroxy or methoxy group, and m is 1 or 2 and n is 0, 1 or 2.

In particularly preferred embodiments of the present invention, the substituents are chosen from a chlorine atom and a group selected from $-CH_2OH$, $-CHO$, $-CH_2OCH_3$, $-CO_2H$, $-CO_2CH_3$, $-CO_2CH_2CH_3$, $-CONH_2$ and $-CONHCH_3$.

A yet further preferred class of compounds of general formula (I) is that wherein $R^1$ represents a hydrogen atom or a halogen atom or a group selected from $C_{1-6}$alkyl, $C_{1-6}$alkoxy or fluoro$C_{1-6}$alkyl, and in particular a hydrogen atom or halogen atom or a $C_{1-3}$alkyl group. Especially preferred are compounds wherein $R^1$ is a bromine atom.

Conveniently, in the compounds of general formula (I), the group Het-$CH_2$— is attached at the 5- or 6-position on the benzthiophen ring, and especially the 5-position.

Also conveniently, in the compounds of general formula (I), $R^3$ may be the group $-CO_2H$, or a C-linked tetrazolyl group. In particular, $R^3$ represents a C-linked tetrazoyl group. Still conveniently, in the compounds of general formula (I), $R^4$ and $R^5$ may each independently represent a hydrogen atom or a halogen atom. In particular $R^4$ and $R^5$ each represent hydrogen atoms.

Particularly preferred compounds of the invention include :

1-[[3-bromo-2-[2-(1H-tetrazol-5-yl)phenyl]-5-benzo[b]thiophenyl]methyl]-2-butyl-4-chloro-1H-imidazole-5-carboxylic acid ;

1-[2-[2-(1H-tetrazol-5-yl)phenyl]-5-benzo[b]thiophenyl]methyl]-2-butyl-4-chloro-1H-imidazole-5-carboxylic acid ;

and physiologically acceptable salts, solvates and metabolically labile esters thereof.

In accordance with the first aspect of the present invention, there is also provided a compound of the general formula (I) above or a physiologically acceptable salt, solvate or metabolically labile ester thereof wherein

$R^1$ represents a hydrogen atom or a halogen atom ;

Ar represents the group

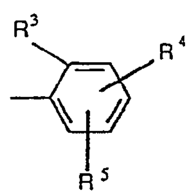

$R^3$ represents a C-linked tetrazolyl group ;

$R^4$ and $R^5$ each independently represent a hydrogen atom ;

Het represents an N-linked imidazolyl group substituted at the 2-position by a $C_{1-6}$alkyl group, the imidazolyl group being substituted by two further substituents selected from a halogen atom or a $-(CH_2)_nCOR^7$ group ;

$R^7$ represents a hydroxy group ; and

n represents an integer from 0 to 4.

The physiologically acceptable acid addition salts of the compounds of formula (I) may be derived from inorganic or organic acids. Examples of such salts include hydrochlorides, hydrobromides, sulphates, phosphates, benzoates, methanesulphonates or trifluoroacetates.

The compounds may also form salts with suitable bases. Examples of such salts are alkali metal (e.g. sodium or potassium), alkaline earth metal (e.g. calcium or magnesium), ammonium and substituted ammonium (e.g. dimethylammonium, triethylammonium, 2-hydroxyethyldimethylammonium, piperazinium, N,N-dimethylpiperazinium, tetralkylammonium, piperidinium, ethylenediammonium and choline).

It will be appreciated that, for pharmaceutical use, the salts referred to above will be physiologically acceptable, but other salts may find use, for example, in the preparation of the compounds of formula (I) and the physiologically acceptable salts thereof.

It will be further appreciated that the compounds of general formula (I) may be chemically modified in the form of compounds which in vivo (for example, by enzymic attack) will provide the parent compounds of general formula (I). Such prodrugs may be, for example, physiologically acceptable metabolically labile ester derivatives. These may be formed by esterification, for example of any of the carboxylic acid groups in the parent compound of general formula (I), with prior protection of any other reactive groups present in the molecule. Examples of such esters include lower alkyl esters (e.g. methyl or ethyl esters), alkenyl esters (e.g. vinyl or alkyl esters), alkynyl esters(e.g. ethynyl or propynyl esters), alkoxyalkyl esters, (e.g. methoxymethyl or 2-methoxyethyl esters), alkylthioalkyl esters (e.g. methylthiomethyl esters) haloalkyl esters (e.g. 2-iodoethyl or 2,2,2,-trichloromethyl esters), alkanoyloxyalkyl esters (e.g. acetoxymethyl, 2-acetoxymethyl or pivaloyloxymethyl esters), alkoxycarbonyloxyalkyl esters (e.g. 1-ethoxycarbonyloxyethyl or 1-methoxycarbonyloxyethyl esters), aroyloxyalkyl esters (e.g. benzoyloxymethyl or 1-benzoyloxyethyl esters), substituted or unsubstituted aralkyl esters (e.g. benzyl or 4-amidobenzyl esters), substituted or unsubstituted aminoethyl esters (e.g aminoalkyl or 2-N,N-dimethylaminoethyl esters) or hydroxyalkyl esters (e.g. 2-hydroxyethyl or 2,3-dihydroxypropyl esters).

In addition to the above ester derivatives the present invention includes within its scope compounds of general formula (I) in the form of other physiologically acceptable equivalents, i.e. physiologically acceptable compounds which, like the metabolically labile esters, are converted in vivo into the parent compounds of general formula (I).

According to a second aspect of the present invention we provide a compound of formula (I) or a physiologically acceptable salt, solvate or metabolically labile ester thereof for use in therapy.

In particular, the compounds of the invention may be used in the treatment or prophylaxis of hypertension. They are also potentially useful for the treatment of cognitive disorders such as dementia (e.g. Alzheimer's disease) and other diseases such as renal failure, hyperaldosteronism, cardiac insufficiency, congestive heart failure, post-myocardial infarction, cerebrovascular disorders, glaucoma and disorders of intracellular homeostasis.

According to a further aspect of the present invention we provide a compound of formula (I) or a physiologically acceptable salt, solvate or metabolically labile ester thereof for use in the treatment of the aforementioned diseases, especially hypertension.

According to another aspect of the present invention we provide a compound of formula (I) or a physiologically acceptable salt, solvate or metabolically labile ester solvate thereof for the manufacture of a therapeutic agent for the treatment of the aforementioned diseases, especially hypertension.

According to a further aspect of the present invention we provide a method of treating the aforementioned diseases, especially hypertension, which method comprises administering an effective amount to a patient in need of such treatment of a compound of formula (I) or a physiologically acceptable salt, solvate or metabolically labile ester thereof.

It will be appreciated that the compounds of formula (I) or a physiologically acceptable salt, solvate, or metabolically labile ester thereof may advantageously be used in conjunction with one or more other therapeutic agents, such as for example diuretics and/or different antihypertensive agents such as β-blockers, calcium channel blockers or ACE inhibitors. It is to be understood that such combination therapy constitutes a further aspect of the present invention.

It will be further appreciated that reference herein to treatment extends to phrophylaxis as well as to the treatment and relief of established symptoms.

While it is possible that a compound of general formula (I) may be administered as the raw chemical it is preferable to present the active ingredient as a pharmaceutical formulation.

The compounds of formula (I) and their physiologically acceptable salts, solvates and metabolically labile esters may be formulated for administration in any convenient way, and the invention also includes within its scope pharmaceutical compositions comprising at least one compound of formula (I) or a physiologically acceptable salt, solvate or metabolically labile ester thereof adapted for use in human or veterinary medicine. Such compositions may be presented for use in conventional manner in admixture with one or more physiologically acceptable carriers or excipients. The carrier(s) must be 'acceptable' in the sense of being compatible

with the other ingredients of the formulation and not deleterious to the recipient thereof.

Thus, the compounds according to the invention may be formulated for oral, buccal, parenteral or rectal administration or in a form suitable for administration by inhalation or insufflation. Oral administration is preferred.

Tablets and capsules for oral administration may contain conventional excipients such as binding agents, for example mucilage of starch or polyvinylpyrrolidone ; fillers, for example, lactose, microcrystalline cellulose or maize-starch ; lubricants, for example, magnesium stearate or stearic acid ; disintegrants, for example, potato starch, croscarmellose sodium or sodium starch glycollate ; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup or carboxymethyl cellulose ; emulsifying agents, for example, sorbitan mono-oleate ; nonaqueous vehicles (which may include edible oils), for example, propylene glycol or ethyl alcohol ; and preservatives, for example, methyl or propyl p-hydroxybenzoates or sorbic acid. The compounds or their salts or esters may also be formulated as suppositories, e.g. containing conventional suppository bases such as cocoa butter or other glycerides. For buccal administration the composition may take the form of tablets or lozenges formulated in conventional manner.

It will be appreciated that both tablets and capsules may be manufactured in the form of sustained release formulations, such that they provide a controlled continuous release of the compounds according to the invention over a period of hours.

The compounds of formula (I) and their physiologically acceptable salts, solvates and metabolically labile esters may be formulated for parenteral administration by bolus injection or continuous infusion and may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoro methane, dichlorotetrafluoroethane or other suitable gas. In the case of a pressurised aerosol the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The pharmaceutical formulations according to the invention may also contain other active ingredients such as antimicrobial agents, or preservatives.

It will be appreciated that the amount of a compound of general formula (I) required for use in treatment will vary not only with the particular compound selected but also with the route of administration, the nature of the condition being treated and the age and condition of the patient and will ultimately be at the discretion of the attendant physician or veterinarian. In general, however, when the compositions comprise dosage units, each unit will preferably contain 5 mg to 500 mg, advantageously where the compounds are to be administered orally 25 mg to 400 mg of the active compound. The daily dosage as employed for adult human treatment will preferably range from 5 mg to 3 g, most preferably from 25 mg to 1 g which may be administered in 1 to 4 daily doses.

The compounds of the invention may be prepared by a number of processes as described below wherein the various groups are as defined for general formula (I) unless otherwise specified.

Thus, according to a further aspect of the present invention we provide a process (A) for preparing the compounds of general formula (I) which comprises treating a compound of general formula (II)

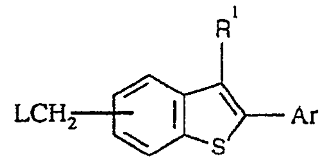

(II)

(wherein L is a leaving group, for example a halogen atom such as chlorine, bromine or iodine, or a hydrocarbylsulphonyloxy group such as methanesulphonyloxy, or p-toluenesulphonyloxy and $R^1$ and Ar are as defined in general formula (I)) with an imidazole of formula (III)

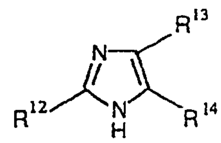

(III)

(wherein $R^{12}$ represents a hydrogen atom or a group selected from $C_{1-6}$alkyl, $C_{2-6}$alkenyl or $C_{1-6}$alkylthio ; $R^{13}$ and $R^{14}$ which may be the same or different each independently represent a hydrogen or halogen atom, or a group selected from cyano, nitro, $C_{1-6}$alkyl, $C_{2-6}$alkenyl, fluoro$C_{1-6}$alkyl, $-(CH_2)_mR^6$, $-(CH_2)_nCOR^7$, $-(CH_2)_pNR^8COR^9$ ; and $R^6$, $R^7$, $R^8$, $R^9$, m, n and p are as defined in general formula (I)) followed by the removal of any protecting groups where present, as described hereinafter.

The reaction is preferably effected under basic conditions, for example, in the presence of sodium hydride, potassium carbonate or sodium methoxide. The reaction is conveniently effected in a solvent such as acetonitrile or an ether e.g. tetrahydrofuran or dioxan, a ketone e.g. butanone or methyl isobutyl ketone, or a substituted amide e.g. dimethylformamide, at a temperature between 0°C and the reflux temperature of the solvent.

The intermediate compounds of general formula (II) and their acid addition salts are novel compounds and form a further aspect of the present invention.

In another general process (B) a compound of general formula (I) may be obtained by deprotection of a protected intermediate of general formula (IV)

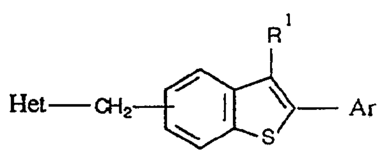

(IV)

(wherein $R^1$, Ar and Het are as defined in general formula (I) except that at least one reactive group is blocked by a protecting group).

The protecting groups may be any conventional protecting groups, for example as described in "Protective Groups in Organic Synthesis" by Theodora Greene (John Wiley and Sons Inc., 1981). Examples of carboxyl protecting groups include $C_{1-6}$ alkyl such as methyl or t-butyl, or $C_{7-10}$ aralkyl such as benzyl.

When $R^3$ is a tetrazole group, this may be protected with, for example, the trityl group $-C(phenyl)_3$, or a p-nitrobenzyl or 1-ethoxyethyl group.

Deprotection to yield the compound of general formula (I) may be effected using conventional techniques. Thus, for example, aralkyl groups may be cleaved by hydrogenolysis in a suitable organic solvent such as an alcohol, e.g. ethanol, in the presence of a noble metal catalyst such as palladium or platinum or an oxide thereof on a support such as charcoal, and conveniently at room temperature and pressure. Carboxyl protecting groups such as alkyl groups may be cleaved by hydrolysis using a base such as an alkali metal hydroxide (e.g. sodium hydroxide or potassium hydroxide) in a suitable solvent (e.g. an aqueous alcohol such as methanol or ethanol) at any suitable temperature up to reflux. Deprotection of the tetrazole group when protected with a trityl group may be effected by acid hydrolysis using trifluoroacetic acid or a mineral acid such as hydrochloric acid in a suitable solvent such as ethanol conveniently at room temperature. Alternatively, when possible, deprotection of the tetrazolyl group can be effected by catalytic hydrogenation as previously described.

In another general process (C) a compound of general formula (I) in which the substituent $R^3$ in the group Ar represents a C-linked tetrazolyl group (and the imidazolyl group represented by Het is not substituted by a

cyano group), may also be prepared from a compound of general formula (Ia)

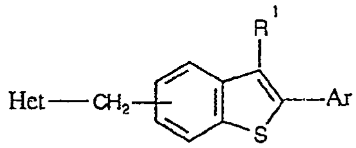

(Ia)

(wherein $R^1$, Ar and Het are as defined in general formula (I) except that in the group Ar, $R^3$ represents a nitrile group) by reaction with a suitable azide such as sodium azide, ammonium azide (preferably prepared in situ from sodium azide and ammonium chloride) or tributyl tin azide. The reaction is conveniently effected in a solvent such as xylene at an elevated temperature, such as the reflux temperature of the solvent, for between 1 and 10 days. Where the azide is tributyl tin azide the reaction may conveniently be effected in the absence of a solvent at a temperature between room temperature and 180°C. Such a reaction leaves the tetrazolyl group protected with a tributyl tin group, which can readily be removed using aqueous base or acid. Where aqueous base is used to effect this deprotection, the compound may be treated with an aqueous acid to liberate the free acid.

Compounds of general formula (Ia) may be prepared by processes analogous to those described herein commencing from a compound of formula (VIII) and a corresponding benzthiophen intermediate.

The intermediate compounds of general formula (Ia) and their acid addition salts are novel compounds and form a further aspect of the present invention.

In another general process (D) a compound of general formula (I) in which the substituent $R^3$ in the group Ar represents $—NHSO_2CF_3$, may also be prepared from a compound of general formula (Ib)

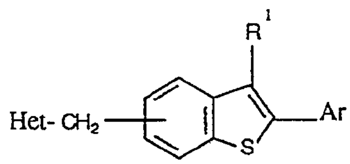

(Ib)

(wherein $R^1$, Ar and Het are as defined in general formula (I) except that in the group Ar, $R^3$ represents an amino group) by reaction with trifluoromethanesulphonic anhydride, in a suitable solvent such as dichloromethane.

Compounds of general formula (Ib) may be prepared by processes analogous to those described herein commencing from a compound of formula (IX) and a corresponding benzthiophen intermediate.

Alternatively, compounds of general formula (Ib) may be prepared by a Curtius rearrangement of a compound of formula (I) wherein $R^3$ in the group Ar is $—CO_2H$ (provided that this is the only carboxyl group in the molecule) using, for example, diphenylphosphorylazide in the presence of a base such as triethylamine and in a solvent such as an alcohol (e.g. tert-butanol) to form a carbonate followed by deprotection of the amine in a conventional manner, for example by acid hydrolysis using hydrochloric acid in a solvent such as ethanol.

The intermediate compounds of general formula (Ib) and their acid addition salts are novel compounds and form a further aspect of the present invention.

In the processes (A), (B), (C) and (D) described above, the compounds of general formula (I) may be obtained in the form of a salt, conveniently in the form of a physiologically acceptable salt. Where desired, such salts may be converted into the corresponding free acids or free bases using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or isopropanol.

Physiologically acceptable salts may also be prepared from other salts, including other physiologically acceptable salts, of the compounds of general formula (I), using conventional methods.

The intermediate compounds of general formula (II) may be prepared from a compound of formula (V) :

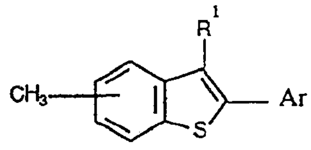

(V)

using any suitable reagent well known in the art for converting the methyl on the 6-membered ring into the group $-CH_2L$ (wherein L is as defined above). Thus, for example, when L is a halogen atom, a compound of formula (V) can be converted into a compound of general formula (II) using N-chloro amides, tert-butyl hypochlorite or N-bromosuccinimide. Halogenation of the side chain may be catalysed by light, thus the reaction can be illuminated with a suitable artificial light source, and preferably in the presence of a free radical initiator such as azobisisobutyronitrile (AIBN) or benzoyl peroxide.

Compounds of formula (V) wherein $R^1$ is a halogen atom, for example, a bromine atom, may be prepared by halogenation of a compound of formula (V) wherein $R^1$ represents a hydrogen atom, using for example, bromine, in a suitable solvent such as a halogenated hydrocarbon, e.g. carbon tetrachloride.

Compounds of formula (V) may be prepared by reaction of a compound of formula (VI)

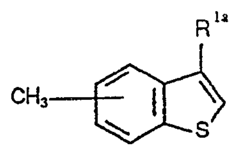

(VI)

(wherein $R^{1a}$ represents a hydrogen atom or a group selected from $C_{1-6}$alkyl or $C_{2-6}$alkenyl) with a compound of formula (VII)

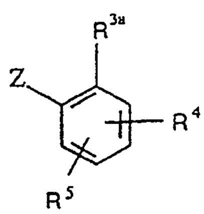

(VII)

(wherein Z represents a bromine or iodine atom or the group $-OSO_2CF_3$, $R^4$ and $R^5$ are as defined in general formula (I) and $R^{3a}$ is as defined for $R^3$ in general formula (I) or is a protected derivative thereof).

The compound of formula (VI) is first treated with an alkyl lithium compound such as n-butyl lithium at a reduced temperature, for example, between -100°C and 0°C in a solvent such as an ether (e.g. tetrahydrofuran). The mixture is then treated with a tri-alkylborate compound such as triisopropylborate and the temperature conveniently brought up to room temperature. Subsequently, water may be added and the mixture treated with a mineral acid such as sulphuric acid thus producing a compound of formula (VIa)

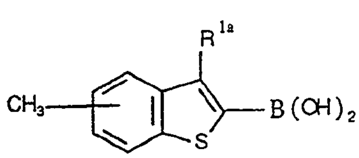

(VIa)

The intermediate compound of formula (VIa) is then reacted with a compound of formula (VII) in the presence of a palladium (0) compound such as tetrakis(triphenylphosphine) palladium (0) in a solvent such as an ether (e.g. dimethoxyethane), and in the presence of a base such as sodium carbonate or thallium hydroxide. The reaction is conveniently effected at an elevated temperature, such as the reflux temperature of the solvent.

Compounds of formula (V) in which the substituent $R^3$ in the group Ar represents a C-linked tetrazolyl group may be prepared from a precursor of a compound of formula (V) wherein the substituent $R^3$ represents a nitrile group using the reagents and conditions described in process (C).

Similarly, intermediates of formula (VII) wherein $R^{3a}$ represents a C-linked tertazolyl group may be prepared from a compound of formula (VIII)

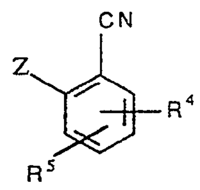

(VIII)

(followed where necessary by protection of any reactive groups), using methods well-known in the art such as those described in process (C).

Compounds of formula (V) in which the substituent $R^3$ in the group Ar is —$NHSO_2CF_3$ may be prepared from a precursor of a compound of formula (V) wherein the substituent $R^3$ is an amine group using the reagents and conditions described in process (D).

Similarly, intermediates of formula (VII) wherein $R^{3a}$ represents—$NHSO_2CF_3$ may be prepared from a compound of formula (IX),

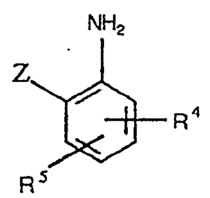

(IX)

(followed where necessary by the protection of any reactive group) using methods well known in the art such as those described in process (D).

Compounds of formula (V) may also be prepared by an intramolecular cyclisation reaction of a compound of formula (X)

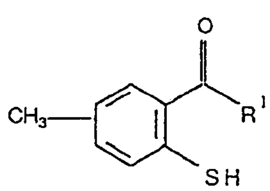

(X)

(wherein $R^1$ is as previously defined) with a suitably substituted benzene of formula (XI)

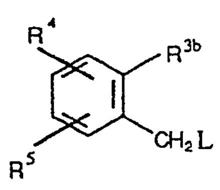

(XI)

(wherein L is as previously defined and $R^{3b}$ is as defined for $R^{3a}$ in formula (VII) or is a nitrile group suitable for subsequent conversion into a tetrazolyl group or is an amino group suitable for conversion into—$NHSO_2CF_3$), in the presence of a base such as sodium hydride or potassium carbonate. The cyclisation is a two step reaction which requires one equivalent of base per step. It will be appreciated however that the reaction can be effected in the presence of two equivalents of base to avoid the need to isolate the intermediate. The reaction is conveniently effected in a solvent such as an ether e.g tetrahydrofuran, an alcohol e.g ethanol or a substituted amide e.g dimethylformamide, at a temperature between room temperature and the reflux temperature of the solvent.

It will be appreciated that compounds of formula (V) in which $R^1$ represents a hydrogen or halogen atom may also be converted into compounds of formula (V) in which $R^1$ represents the group methyl (via hydrogenolysis of the Mannich base), —CHO or —$COR^2$ (wherein $R^2$ is as defined in general formula (I)) using techniques well known in the art, such as those described in "Heterocyclic Chemistry" by J.A. Joule and G.F. Smith, Van Nostrand Reinhold Company, London (1972), "Heterocyclic Chemistry" by A. Albert, 2nd Edition, The Athlone Press, London (1968), "Heterocyclic Compounds", Vol. 29 by A. Mustafa, John Wiley and Sons Inc., New York (1974), "Heterocyclic Compounds", Vol. 2 by R.C. Elderfield, John Wiley and Sons Inc., New York (1951) and "Advances in Heterocyclic Chemistry", Vol. 29 by A.R. Katritsky and A.J. Boulton, Academic Press, New York (1981).

The imidazoles of formula (III) may be prepared as described in European Specification No. 0253310A and in US Patent No. 4355040 or by methods analogous to those described therein. The content of these references is hereby incorporated by reference.

Intermediates of formulae (VI), (VII), (VIII), (IX), (X) and (XI), are either known compounds or may be prepared by methods analogous to those used for the preparation of the known compounds.

The following examples illustrate the invention. Temperatures are in °C. "Dried" refers to drying using magnesium sulphate. Thin layer chromatography (t.l.c.) was carried out over silica and column chromatography was carried out on silica (Merck 9385 unless otherwise stated), using ether or the following solvent systems : A - ether : hexane. The following abbreviations are used : THF - tetrahydrofuran ; DME - dimethoxyethane ; DMF - dimethylformamide ; NBS - N-bromosuccinimide ; BPO - benzoyl peroxide.

Intermediate 1

2-Butyl-4-chloro-1H-imidazole-5-carboxaldehyde

A suspension of 2-butyl-4-chloro-1H-imidazole-5-methanol (22.0 g) in dichloromethane : 1,4-dioxan (2 : 1) (690 ml) was treated with manganese dioxide (63.15 g) and the reaction mixture heated at reflux under nitrogen for 3.5 h. The reaction mixture was cooled, filtered and the filtrate evaporated to leave an off-white solid. The residue was triturated with petroleum ether, filtered and dried to give the title compound as a white solid (17.9 g) m.p. 98-99°C.

Intermediate 2

2-(5-Methyl-2-benzthiophenyl)boronic acid

A solution of 5-methylbenzthiophene (5 g) in dry ether was cooled to 0°C under nitrogen and treated dropwise with n-butyl lithium (22.2 ml) maintaining the temperature below +5°C. After a further 1 h at 0°C the reaction was cooled to -50°C and treated with triisopropylborate (23.4 ml). The reaction was warmed gradually to room temperature overnight. 2 N HCl was added to the solid so formed. The organic phase was separated and washed with further 2 N HCl and water before drying. Filtration and evaporation gave a yellow solid. Trituration with hexane gave the title compound as a white powder (4.54 g).
T.l.c. ether Rf 0.8

Intermediate 3

2-(5-Methyl-2-benzthiophenyl)benzonitrile

A solution of 2-bromobenzonitrile (1.71 g) in DME and 2 N sodium carbonate was heated to reflux under nitrogen with vigorous stirring before addition of Intermediate 2 (2 g) and tetrakistriphenylphosphine palladium (0.5 g). Heating at reflux under nitrogen was continued overnight. The reaction was cooled to room temperature and the reaction diluted with ether and washed with water. The water was back extracted with ethyl acetate and the combined organic extracts dried, filtered and evaporated to give a brown oil. Crystallisation from methanol (200 ml) gave a brownish solid. The solid so obtained was suspended in dichloromethane (25 ml) filtered through a pad of silica eluting with further dichloromethane and evaporated to give the title compound as a white solid (1.28 g).
T.l.c. System A (1 : 9) Rf 0.4

Intermediate 4

2-[3-Bromo-5-methyl-2-benzothiophenyl]benzonitrile

A suspension of Intermediate 3 (0.5 g) in trichloromethane was treated with sodium acetate (0.325 g) and bromine (0.115 ml). Stirring at room temperature in the dark was continued overnight. The reaction was poured into water and the organic phase diluted with dichloromethane. The organic phase was separated and washed with 2 N NaOH and water and dried. Filtration and evaporation gave a pale yellow oil (0.585 g). Purification by column chromatography eluting with System A (1 : 9) gave the title compound as a white solid (0.443 g).
T.l.c. System A (1 : 9) Rf 0.35.

Intermediate 5

2-[[3-Bromo-5(bromomethyl)-2-benzo[b]thiophenyl]benzonitrile

A solution of Intermediate 4 (1.1 g) in carbon tetrachloride was treated with NBS (0.656 g) and BPO (~100 mg) and heated to reflux whilst irradiating with a 250 W tungsten bulb. After 1 hr the reaction was cooled to room temperature and filtered. The solution was diluted with dichloromethane and washed with water. The organic phase was dried, filtered and evaporated to give the title compound as a white foam (0.94 g).
T.l.c. System A (1 : 9) Rf 0.35.

Intermediate 6

2-[5-[2-Butyl-4-chloro-5-(oxomethyl)-1H-imidazol-1-yl]methyl-3-bromo-2-benzothiophenyl]benzonitrile

A solution of Intermediate 5 (1.24 g) in dry DMF was treated with Intermediate 1 (0.565 g) and potassium carbonate (0.44 g) and heated to 80°C under nitrogen. After 4 h the solvent was removed under reduced pressure and the residue partitioned between ethyl acetate and water. The phase organic phase was washed with water and the aqueous phase back extracted with ethyl acetate. The combined organic extracts were dried, filtered and evaporated to give an orange oil (1.47 g). Purification by column chromatography, eluting with System A (1 : 1) gave the title compound as a pale yellow foam (0.64 g).
T.l.c. System A (1 : 1) Rf 0.35

Intermediate 7

1-[[3-Bromo-2-(2-cyanophenyl)-5-benzo[b]thiophenyl]methyl]-2-butyl-4-chloro1H-imidazole-5-carboxylic acid

A solution of Intermediate 6 (10.62 g) in THF, tert-butanol and 2- methylbut-2-ene (7.26 ml) was heated with a solution of sodium chlorite (1.09 g) and sodium dihydrogen phosphate (1.09 g) in water. Stirring at room temperature was continued overnight. The solvent volume was reduced and the residue portioned between ethyl acetate and 2NHCl. The organic phase was separated and the aqueous phase extracted with further ethyl acetate. The combined organic extracts were washed with water, dried, filtered and evaporated to give the title compound as an off-white foam (0.746 g).
T.l.c. ether Rf 0.4

Intermediate 8

2-[5-(Bromomethyl)-2-benzthiophenenyl]benzonitrile

A solution of Intermediate 3 (1 g) in dry carbon tetrachloride was treated with NBS (0.79 g) and BPO (~100 mg) according to the method of Intermediate 5 to give the title compound as a yellow oil which crystallised on standing (1.39 g)
T.l.c. System A (1 : 9) Rf 0.37

Intermediate 9

2-[5-[2-Butyl-4-chloro-5-(oxomethyl)-1H-imidazol-1-yl]methyl-2-benzthiophenyl]benzonitrile

A solution of Intermediate 8 (1.3 g) in dry DMF was treated with Intermediate 1 (0.74 g) and potassium carbonate according to the method of Intermediate 6 to give the title compound as an orange oil (0.634 g).
T.l.c. System A (1 : 1) Rf 0.3

Intermediate 10

1-[[2-(2-Cyanophenyl)-5-benzo[b]thiophenyl]methyl]-2-butyl-4-chloro-1H-imidazole-5-carboxylic acid

A solution of sodium chlorite (1.29 g) and sodium dihydrogen phosphate (1.29 g) in water was added to Intermediate 9 (0.62 g) in tert-butanol, 2-methylbut-2-ene (8.58 ml) and THF. Stirring at room temperature was continued overnight. The solvent volume was reduced and the residue portioned between 1 N NaOH and ether. The mixture was acidified to ~pH 3 and the organic phase separated. The aqueous phase was further extracted with ethyl acetate. The combined organic extracts were dried, filtered and evaporated to give the title compound as a pale yellow powder (0.61 g).
T.l.c. ether, Rf 0.4

Example 1

1-[3-Bromo-2-[2-(1H-tetrazol-5-yl)phenyl]-5-benzo[b]thiophenyl]methyl] -2-butyl-4-chloro-1H-imidazole-5-carboxylic acid

A suspension of Intermediate 7 (0.64 g) in n-butyl tin azide (5 g) was heated under nitrogen at 160°C for 1 h. The reaction was cooled and diluted with 0.5 M NaOH. The resulting solution was washed with ether before acidification to pH~2 using 2 N HCl. The suspension so formed was extracted with ethyl acetate and the combined organic extracts dried, filtered and evaporated to give a pale yellow foam. Trituration with hexane gave a powdered solid (0.52 g) which was further purified by repeating the workup procedure to give the title compound as a yellow powder (0. 302 g).
m.p. 144-146°C
H.p.l.c Dynamax -60A Solvent A : $H_2O$ (0.05% TFA), Solvent B : acetonitrile : $H_2O$ (1 : 1 + 0.05% TFA)
Retention time = 23.52 minutes

Example 2

1-[2-[2-(1H-tetrazol-5-yl)phenyl]-5-benzo[b]thiophenyl]methyl]-2 butyl-4-chloro-1H-imidazole-5-carboxylic acid

A suspension of Intermediate 10 (0.60 g) in n-butyl tin azide (5 g) was heated under nitrogen. Heating at 160°C was continued for 1 h. The reaction was cooled and diluted with 0.5 M NaOH. The resulting solution was washed with ether before acidification to pH2 using 2 N HCl. The suspension so formed was extracted with ethyl acetate and the combined organic extracts dried, filtered and evaporated to give a pale yellow foam (0.38 g). This was triturated with ether to give a yellow solid (0.183 g). Material crystallised from the mother liquors giving the title compound as pale yellow crystals (0.104 g).
m.p. 157-159°C
H.p.l.c Retention = time 22.26 minutes

The compounds of the invention are tested in vitro for angiotensin II antagonism. Aortic strips are obtained from male New Zealand white rabbits and prepared for recording isometric contractions in response to cumulative addition of angiotensin II. The potencies of test antagonists are assessed by measuring their abilities to displace the angiotensin II cumulative concentration response curve. The method used is that of Ackerly et al., Proc. Natl. Acad. Sci., 74 (12), pp5725-28 (1977) with the exception that the final composition of the physiological salt solution is as given below in the Table

TABLE

| Ingredient | Amount (mM) |
|---|---|
| $Na^+$ | 143.4 |
| $K^+$ | 5.9 |
| $Mg^{2+}$ | 0.6 |
| $Ca^{2+}$ | 1.3 |
| $Cl^-$ | 124.5 |
| $HPO_4^-$ | 1.2 |
| $SO_4^{2-}$ | 0.6 |
| $HCO_3^-$ | 25.0 |
| glucose | 11.1 |
| indomethacin | 0.005 |
| ascorbic acid | 0.1 |

The tissues are initially challenged with $K^+$ (80 mM) and then washed at 0, 5, 10 and 15 minutes after the response to $K^+$ has plateaued. After a further 45 minutes an angiotensin II cumulative response curve is constructed (0.1 nM to 0.1 µM in 10-fold increments) and the tissues are washed as before. A second, third and fourth angiotensin II cumulative response curve (0.1 nM to 0.1 µM in 3-fold increments) is then constructed at hourly intervals (15 minutes washing after each curve followed by 45 minutes equilibration). The compounds of the invention (30 µM) are tested for angiotensin II antagonism by application 45 minutes before construction of the fourth angiotensin II curve. The third and fourth angiotensin II curves are expressed graphically and a concentration ratio (CR) is calculated by dividing the angiotensin II $EC_{50}$ value obtained in the presence of the test antagonist (i.e. fourth curve) by the angiotensin II $EC_{50}$value obtained in the absence of the test antagonist (i.e. third curve).

The potency of the test antagonist is expressed as a pKb which is calculated from the equation :

$$PkB = -Log\left[\frac{CR-1}{[antagonist]}\right]$$

which is a rearrangement of equation 4 described by Furchgott, in Handbook of Exp. Pharmacol., 33, p290 (1972) (eds. Blaschkott and Muscholl).

Compounds of the invention will desirably exhibit a pKb in the range between 5 and 12. Thus we have found that the compounds of the invention inhibit the action of the hormone angiotensin II and are therefore useful in the treatment of conditions in which it is desirable to inhibit angiotensin II activity. In particular, the compounds of the Examples have been tested in the above test and have been found to be active.

There is thus provided as a further aspect of the present invention a compound of general formula (I) or a physiologically acceptable salt, solvate or metabolically labile ester thereof for use in the treatment of conditions associated with excessive or unregulated angiotensin II activity.

In a further or alternative aspect of the present invention there is provided a compound of general formula (I) or a physiologically acceptable salt, solvate or metabolically labile ester thereof for the manufacture of a therapeutic agent for the treatment of conditions associated with excessive or unregulated angiotensin II activity.

There is also provided in a further or alternative aspect of the present invention a method for the treatment of conditions associated with excessive or unregulated angiotensin II activity in a mammal including man comprising administration of an effective amount to a mammal in need of such treatment of a compound of general formula (I) or a physiologically acceptable salt, solvate or a metabolically labile ester thereof.

The following examples illustrate pharmaceutical formulations according to the invention. The term "active ingredient" is used herein to represent a compound of formula (I).

Pharmaceutical Example 1

Oral Tablet A

| | |
|---|---|
| Active Ingredient | 700mg |
| Sodium starch glycollate | 10mg |
| Microcrystalline cellulose | 50mg |
| Magnesium stearate | 4mg |

Sieve the active ingredient and microcrystalline cellulose through a 40 mesh screen and blend in a appropriate blender. Sieve the sodium starch glycollate and magnesium stearate through a 60 mesh screen, add to the powder blend and blend until homogeneous. Compress with appropriate punches in an automatic tablet press. The tablets may be coated with a thin polymer coat applied by the film coating techniques well known to those skilled in the art. Pigments may be incorporated in the film coat.

Pharmaceutical Example 2

Oral Tablet B

| | |
|---|---|
| Active Ingredient | 500mg |
| Lactose | 100mg |
| Maize Starch | 50mg |
| Polyvinyl pyrrolidone | 3mg |
| Sodium starch glycollate | 10mg |
| Magnesium stearate | 4mg |
| Tablet Weight | 667mg |

Sieve the active ingredient, lactose and maize starch through a 40 mesh screen and blend the powders in a suitable blender. Make an aqueous solution of the polyvinyl pyrrolidone (5-10% w/v). Add this solution to the blended powders and mix until granulated ; pass the granulate through a 12 mesh screen and dry the granules in a suitable oven or fluid bed dryer. Sieve the remaining components through a 60 mesh screen and blend them with the dried granules. Compress, using appropriate punches, on an automatic tablet press.

The tablets may be coated with a thin polymer coat applied by film coating techniques well known to those skilled in art. Pigments may be incorporated in the film coat.

Pharmaceutical Example 3

Inhalation Cartridge

| | |
|---|---|
| Active Ingredient | 1mg |
| Lactose | 24mg |

Blend active ingredient, particle size reduced to a very fine particle size (weight mean diameter ca. 5 μm) with the lactose in a suitable powder blender and fill the powder blender into No. 3 hard gelatin capsules.

The contents of the cartridges may be administered using a powder inhaler.

Pharmaceutical Example 4

Injection Formulation

| | % w/v |
|---|---|
| Active ingredient | 1.00 |
| Water for injections B.P. to | 100.00 |

Sodium chloride may be added to adjust the tonicity of the solution and the pH may be adjusted to that of maximum stability and/or to facilitate solution of the active ingredient using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

## Claims

1. A compound of the general formula (I)

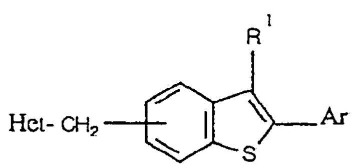

(I)

or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy, —CHO, —$CO_2H$ or —$COR^2$ ;
Ar represents the group

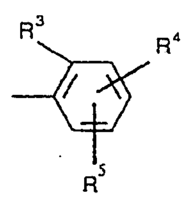

;

$R^2$ represents a group selected from $C_{1-6}$alkyl, $C_{2-6}$alkenyl, $C_{1-6}$alkoxy or the group —$NR^{10}R^{11}$ ;
$R^3$ represents a group selected from —$CO_2H$, —$NHSO_2CF_3$ or a C-linked tetrazolyl group ;
$R^4$ and $R^5$, which may be the same or different, each independently represent a hydrogen atom or a halogen

atom or a $C_{1-6}$alkyl group ;
Het represents an N-linked imidazolyl group optionally substituted at the 2-position by a $C_{1-6}$alkyl, $C_{2-6}$alkenyl or a $C_{1-6}$alkylthio group, the imidazolyl group optionally being substituted at the 4- and 5-positions by one or two further substituents selected from a halogen atom or a group selected from cyano, nitro, $C_{1-6}$alkyl, $C_{2-6}$alkenyl, fluoro$C_{1-6}$alkyl, $-(CH_2)_mR^6$, $-(CH_2)_nCOR^7$ or $-(CH_2)_pNR^8COR^9$ ;
$R^6$ represents a hydroxy or $C_{1-6}$alkoxy group ;
$R^7$ represents a hydrogen atom or a group selected from hydroxy, $C_{1-6}$alkyl, $C_{1-6}$alkoxy, phenyl, phenoxy or the group $-NR^{10}R^{11}$ ;
$R^8$ represents a hydrogen atom or a $C_{1-6}$alkyl group ;
$R^9$ represents a hydrogen atom or a group selected from $C_{1-6}$alkyl, $C_{1-6}$alkoxy, phenyl, phenoxy or the group $-NR^{10}R^{11}$ ;
$R^{10}$ and $R^{11}$, which may be the same or different, each independently represent a hydrogen atom or a $C_{1-4}$alkyl group or $-NR^{10}R^{11}$ forms a saturated heterocyclic ring which has 5 or 6 ring members and may optionally contain in the ring one oxygen atom ;
m represents an integer from 1 to 4 ;
n represents an integer from 0 to 4 ; and
p represents an integer from 1 to 4.

2. A compound as claimed in Claim 1 or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, $-CHO$, $-CO_2H$ or $-COR^2$ ;
Ar represents the group

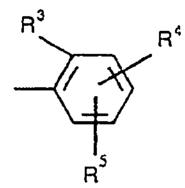

;

$R^2$ represents a group selected from $C_{1-6}$alkyl, $C_{2-6}$alkenyl, $C_{1-6}$alkoxy or the group $-NR^{10}R^{11}$ ;
$R^3$ represents a group selected from $-CO_2H$, $-NHSO_2CF_3$ or a C-linked tetrazolyl group ;
$R^4$ and $R^5$, which may be the same or different, each independently represent a hydrogen atom or a halogen atom or a $C_{1-6}$alkyl group ;
Het represents an N-linked imidazolyl group substituted at the 2-position by a $C_{1-6}$alkyl, $C_{2-6}$alkenyl or a $C_{1-6}$alkylthio group, the imidazolyl group optionnally being substituted by one or two further substituents selected from a halogen atom or a group selected from cyano, nitro, $C_{1-6}$alkyl, $C_{2-6}$alkenyl, perfluoro$C_{1-3}$alkyl, $-(CH_2)_mR^6$, $-(CH_2)_nCOR^7$ or $-(CH_2)_pNR^8COR^9$ ;
$R^6$ represents a hydroxy or $C_{1-6}$alkoxy group ;
$R^7$ represents a hydrogen atom or a group selected from hydroxy, $C_{1-6}$alkyl, $C_{1-6}$alkoxy, phenyl, phenoxy or the group $-NR^{10}R^{11}$ ;
$R^8$ represents a hydrogen atom or a $C_{1-6}$alkyl group ;
$R^9$ represents a hydrogen atom or a group selected from $C_{1-6}$alkyl,$C_{1-6}$alkoxy, phenyl, phenoxy or the group $-NR^{10}R^{11}$ ;
$R^{10}$ and $R^{11}$, which may be the same or different, each independently represent a hydrogen atom or a $C_{1-4}$ alkyl group or $-NR^{10}R^{11}$ forms a saturated heterocyclic ring which has 5 or 6 ring members and may optionally contain in the ring one oxygen atom ;
m represents an integer from 1 to 4 ;
n represents an integer from 0 to 4 ; and
p represents an integer from 1 to 4.

3. A compound as claimed in Claim 1 or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, $C_{1-6}$al-

koxy, —CHO, —$CO_2H$ or —$COR^2$ ;
Ar represents the group

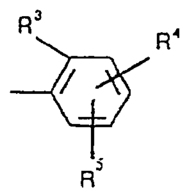

;

$R^2$ represents a group selected from $C_{1-6}$alkyl, $C_{2-6}$alkenyl, $C_{1-6}$alkoxy or the group —$NR^{10}R^{11}$ ;
$R^3$ represents a group selected from —$CO_2H$, —$NHSO_2CF_3$ or a C-linked tetrazolyl group ;
$R^4$ and $R^5$, which may be the same or different, each independently represent a hydrogen atom or a halogen atom or a $C_{1-6}$alkyl group ;
Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or a group selected from cyano, nitro, $C_{1-6}$alkyl, $C_{2-6}$alkenyl, perfluoro$C_{1-3}$alkyl, —$(CH_2)_mR^6$, —$(CH_2)_nCOR^7$ or —$(CH_2)_pNR^8COR^9$ ;
$R^6$ represents a hydroxy or $C_{1-6}$alkoxy group ;
$R^7$ represents a hydrogen atom or a group selected from hydroxy, $C_{1-6}$alkyl, $C_{1-6}$alkoxy, phenyl, phenoxy or the group —$NR^{10}R^{11}$ ;
$R^8$ represents a hydrogen atom or a $C_{1-6}$alkyl group ;
$R^9$ represents a hydrogen atom or a group selected from $C_{1-6}$alkyl, $C_{1-6}$alkoxy, phenyl, phenoxy or the group —$NR^{10}R^{11}$ ;
$R^{10}$ and $R^{11}$, which may be the same or different, each independently represent a hydrogen atom or a $C_{1-4}$alkyl group or —$NR^{10}R^{11}$ forms a saturated heterocyclic ring which has 5 or 6 ring members and may optionally contain in the ring one oxygen atom ;
m represents an integer from 1 to 4 ;
n represents an integer from 0 to 4 ; and
p represents an integer from 1 to 4.

4. A compound as claimed in Claim 1 wherein Het is an N-linked imidazolyl group substituted at the 2-position by a hydrogen atom or a $C_{1-5}$alkyl or a $C_{3-5}$alkenyl group, preferably a $C_{3-5}$alkyl group, for example an n-butyl group, and the N-linked imidazolyl group is substituted at the 4- and 5- positions by one or two further substituents selected from a halogen atom, preferably a chlorine atom, or a group selected from $C_{1-6}$alkyl, —$(CH_2)_mR^6$ or —$(CH_2)_nCOR^7$,especially —$(CH_2)_nCOR^7$, in which $R^7$ is a hydroxy group and n is 0, 1 or 2.

5. A compound as claimed in Claim 4 wherein the N-linked imidazolyl group is substituted at the 4- or 5- position by a —$CO_2H$ group.

6. A compound as claimed in any one of in Claims 1 to 5 wherein the group Het-$CH_2$— is attached at the 5-position on the benzthiophen ring.

7. A compound as claimed in any one of Claims 1 and 4 to 6 wherein $R^1$ represents a hydrogen atom or a halogen atom or a group selected from $C_{1-6}$alkyl, $C_{1-6}$alkoxy or fluoro$C_{1-6}$alkyl, preferably a hydrogen atom or a bromine atom.

8. A compound as claimed in any one of Claims 1 to 7 wherein $R^3$ represents a C-linked tertazolyl group.

9. A compound as claimed in any one of Claims 1 to 8 wherein $R^4$ and $R^5$ each independently represents a hydrogen atom.

10. A compound as claimed in Claim 1 or a physiologically acceptable salt, solvate or metabolically labile ester thereof
wherein
$R^1$ represents a hydrogen atom or a halogen atom ;
Ar represents the group

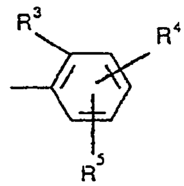 ;

$R^3$ represents a C-linked tetrazolyl group ;
$R^4$ and $R^5$ each independently represent a hydrogen atom ;
Het represents an N-linked imidazolyl group substituted at the 2-position by a $C_{1-6}$alkyl group, the imidazolyl group being substituted at the 4- and 5-positions by two further substituents selected from a halogen atom or a $-(CH_2)_nCOR^7$ group ;
$R^7$ represents a hydroxy group ; and
n represents an integer from 0 to 4.

11. A compound as claimed in Claim 1 selected from :
1-[[3-bromo-2-[2-(1H-tetrazol-5-yl)phenyl]-5-benzo-[b]thiophenyl]methyl]-2-butyl-4-chloro-1H-imidazole-5-carboxylic acid ;
1-[2-[2-(1H-tetrazol-5-yl)phenyl]-5-benzo[b]thiophenyl]methyl]-2-butyl-4-chloro-1H-imidazole-5-carboxylic acid ;
and physiologically acceptable salts, solvates and metabolically labile esters thereof.

12. A process for the preparation of a compound as claimed in any one of Claims 1 to 11 or a physiologically acceptable salt, solvate or metabolically labile ester thereof which comprises :
(A) reacting a benzthiophen of general formula (II)

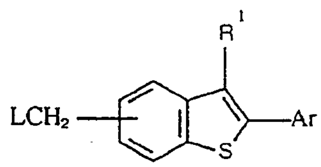 (II)

in which L represents a leaving group, with an imidazole of general formula (III)

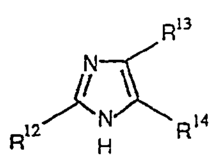 (III)

in which $R^{12}$ represents a hydrogen atom or a group selected from $C_{1-6}$alkyl, $C_{2-6}$alkenyl or $C_{1-6}$alkylthio and $R^{13}$ and $R^{14}$ each independently represent a hydrogen atom or a halogen atom or a group selected from cyano, nitro, $C_{1-6}$alkyl, $C_{2-6}$alkenyl, fluoro$C_{1-6}$alkyl, $-(CH_2)_mR^6$, $-(CH_2)_nCOR^7$, $-(CH_2)_pNR^8COR^9$, followed, if necessary, by removal of any protecting group present ; or
(B) deprotecting a protected intermediate of general formula (IV)

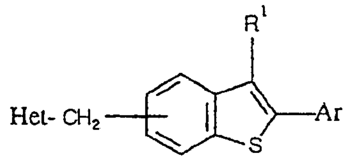

(IV)

in which at least one reactive group is blocked by a protecting group ;

(C) reacting a compound of general formula (Ia)

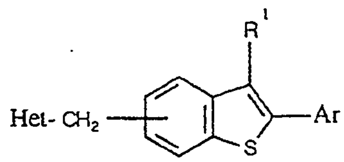

(Ia)

in which $R^3$ in the group Ar represents a nitrile group, with an azide, followed, if necessary, by the removal of any protecting group present ; or

(D) converting a compound of general formula (Ib)

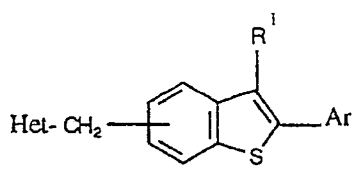

(Ib)

in which $R^3$ in the group Ar represents an amino group, to a compound of general formula (I) in which $R^3$ represents $—NHSO_2CF_3$ ;

and when the compound of formula (I) is obtained as a mixture of enantiomers optionally resolving the mixture to obtain the desired enantiomer ;

and/or, if desired, converting the resulting compounds of general formula (I) or a salt thereof into a physiologically acceptable salt, solvate or metabolically labile ester thereof.

**13.** A process as claimed in Claim 12 which comprises :

(A) reacting a benzthiophen of general formula (II)

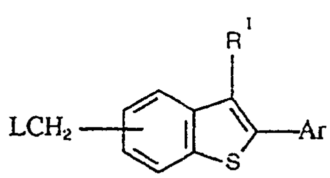

(II)

in which L represents a leaving group, with an imidazole of general formula (III)

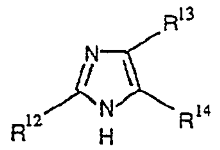

(III)

in which $R^{12}$ represents a hydrogen atom or a group selected from $C_{1-6}$alkyl, $C_{2-6}$alkenyl or $C_{1-6}$alkylthio and $R^{13}$ and $R^{14}$ each independently represent a hydrogen atom or a halogen atom or a group selected from cyano, nitro, $C_{1-6}$alkyl, $C_{2-6}$alkenyl, fluoro$C_{1-6}$alkyl, $-(CH_2)_mR^6$, $-(CH_2)_nCOR^7$, $-(CH_2)_pNR^8COR^9$, followed, if necessary by removal of any protecting group present ; or
(B) deprotecting a protected intermediate of general formula (IV)

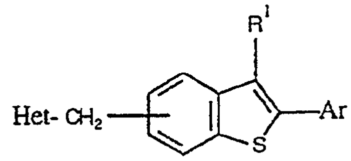

(IV)

in which at least one reactive group is blocked by a protecting group ; or
(C) reacting a compound of general formula (Ia)

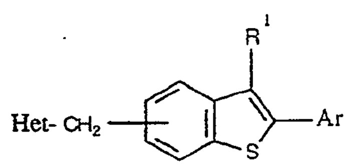

(Ia)

in which $R^3$ in the group Ar represents a nitrile group, with an azide, followed, if necessary by the removal of any protecting group present ;
and when the compound of formula (I) is obtained as a mixture of enantiomers optionally resolving the mixture to obtain the desired enantiomer ;
and/or, if desired, converting the resulting compounds of general formula (I) or a salt thereof into a physiologically acceptable salt, solvate or metabolically labile ester thereof.

**14.** A pharmaceutical composition comprising at least one compound of general formula (I) as defined in any one of claims 1 to 11 or a physiologically acceptable salt, solvate or metabolically labile ester thereof, together with at least one physiologically acceptable carrier or excipient.

**15.** A compound of general formula (I) as claimed in any one of Claims 1 to 11 or a physiologically acceptable salt, solvate or metabolically labile ester thereof for use in therapy, for example,
(i) for use in the treatment or prophylaxis of hypertension ; or
(ii) for use in the treatment or prophylaxis of a disease associated with the cognitive disorders, renal failure, hyperaldosteronism, cardiac insufficiency, congestive heart failure, post-myocardial infarction, cerebrovascular disorders, glaucoma and disorders of intracellular homeostasis ; or
(iii) for use in the treatment of conditions associated with excessive or unregulated angiotensin II activity.

**16.** A compound of general formula (II)

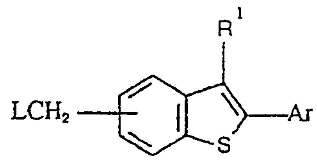

(II)

or an acid addition salt thereof
wherein
$R^1$ and Ar are as defined in Claim 1 ; and
L represents a leaving group.

**17.** A compound of general formula (Ia)

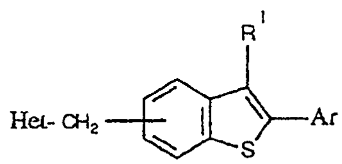

(Ia)

or an acid addition salt thereof
wherein
$R^1$, Ar and Het are as defined in Claim 1 except that in the group Ar, $R^3$ represents a nitrile group.

**18.** A compound of general formula (Ib)

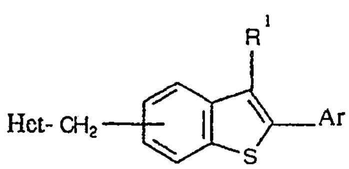

(Ib)

or an acid addition salt thereof
wherein
$R^1$, Ar and Het are as defined in Claim 1 except that in the group Ar, $R^3$ represents an amino group.

**Claims for the following Contracting States : ES & GR**

**1.** A process for the preparation of a compound of the general formula (I)

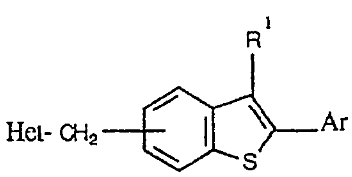

(I)

or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, fluo-

roC$_{1-6}$alkyl, C$_{1-6}$alkoxy, —CHO, —$CO_2H$ or —COR$^2$ ;
Ar represents the group

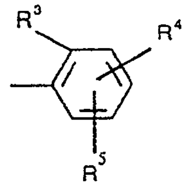
;

$R^2$ represents a group selected from C$_{1-6}$alkyl, C$_{2-6}$alkenyl, C$_{1-6}$alkoxy or the group —NR$^{10}$R$^{11}$ ;
$R^3$ represents a group selected from —$CO_2H$, —$NHSO_2CF_3$ or a C-linked tetrazolyl group ;
$R^4$ and $R^5$, which may be the same or different, each independently represent a hydrogen atom or a halogen atom or a C$_{1-6}$alkyl group ;
Het represents an N-linked imidazolyl group optionally substituted at the 2-position by a C$_{1-6}$alkyl, C$_{2-6}$alkenyl, or a C$_{1-6}$alkylthio group, the imidazolyl group optionally being substituted at the 4- and 5-positions by one or two further substituents selected from a halogen atom or a group selected from cyano, nitro, C$_{1-6}$alkyl, C$_{2-6}$alkenyl, fluoroC$_{1-6}$alkyl, —$(CH_2)_mR^6$, —$(CH_2)_nCOR^7$ or —$(CH_2)_pNR^8COR^9$ ;
$R^6$ represents a hydroxy or C$_{1-6}$alkoxy group ;
$R^7$ represents a hydrogen atom or a group selected from hydroxy, C$_{1-6}$alkyl, C$_{1-6}$alkoxy, phenyl, phenoxy or the group —NR$^{10}$R$^{11}$ ;
$R^8$ represents a hydrogen atom or a C$_{1-6}$alkyl group ;
$R^9$ represents a hydrogen atom or a group selected from C$_{1-6}$alkyl, C$_{1-6}$alkoxy, phenyl, phenoxy or the group —NR$^{10}$R$^{11}$ ;
$R^{10}$ and $R^{11}$, which may be the same or different, each independently represent a hydrogen atom or a C$_{1-4}$alkyl group or —NR$^{10}$R$^{11}$ forms a saturated heterocyclic ring which has 5 or 6 ring members and may optionally contain in the ring one oxygen atom ;
m represents an integer from 1 to 4 ;
n represents an integer from 0 to 4 ; and
p represents an integer from 1 to 4 ;
which comprises :

(A) reacting a benzthiophen of general formula (II)

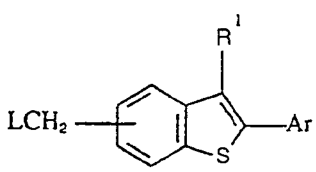
(II)

in which L represents a leaving group, with an imidazole of general formula (III)

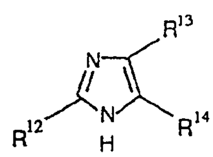
(III)

in which $R^{12}$ represents a hydrogen atom or a group selected from C$_{1-6}$alkyl, C$_{2-6}$alkenyl or C$_{1-6}$alkylthio and $R^{13}$ and $R^{14}$ each independently represent a hydrogen atom or a halogen atom or a group selected from cyano, nitro, C$_{1-6}$alkyl, C$_{2-6}$alkenyl, fluoroC$_{1-6}$alkyl, —$(CH_2)_mR^6$, —$(CH_2)_nCOR^7$, —$(CH_2)_pNR^8COR^9$, followed, if necessary, by removal of any protecting group present ; or

(B) deprotecting a protected intermediate of general formula (IV)

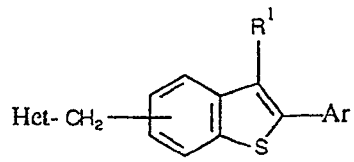

(IV)

in which at least one reactive group is blocked by a protecting group ;
(C) reacting a compound of general formula (Ia)

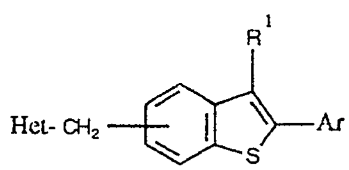

(Ia)

in which $R^3$ in the group Ar represents a nitrile group, with an azide, followed, if necessary, by the removal of any protecting group present ; or
(D) converting a compound of general formula (Ib)

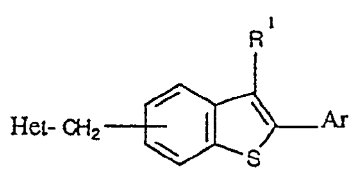

(Ib)

in which $R^3$ in the group Ar represents an amino group, to a compound of general formula (I) in which $R^3$ represents $-NHSO_2CF_3$ ;
and when the compound of formula (I) is obtained as a mixture of enantiomers optionally resolving the mixture to obtain the desired enantiomer ;
and/or, if desired, converting the resulting compounds of general formula (I) or a salt thereof into a physiologically acceptable salt, solvate on metabolically labile ester thereof.

2. A process for the preparation of a compound of general formula (I) as defined in Claim 1 or a physiologically acceptable salt, solvate or metabolically labile ester thereof
which comprises :
(A) reacting a benzthiophen of general formula (II)

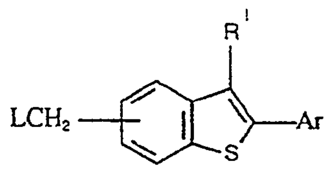

(II)

in which L represents a leaving group, with an imidazole of general formula (III)

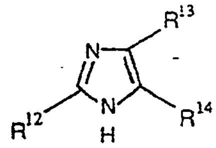

(III)

in which $R^{12}$ represents a hydrogen atom or a group selected from $C_{1-6}$alkyl, $C_{2-6}$alkenyl or $C_{1-6}$alkylthio and $R^{13}$ and $R^{14}$ each independently represent a hydrogen atom or a halogen atom or a group selected from cyano, nitro, $C_{1-6}$alkyl, $C_{2-6}$alkenyl, fluoro$C_{1-6}$alkyl, $-(CH_2)_mR^6$, $-(CH_2)_nCOR^7$, $-(CH_2)_pNR^8COR^9$, followed, if necessary by removal of any protecting group present ; or

(B) deprotecting a protected intermediate of general formula (IV)

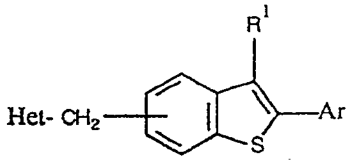

(IV)

in which at least one reactive group is blocked by a protecting group ; or

(C) reacting a compound of general formula (Ia)

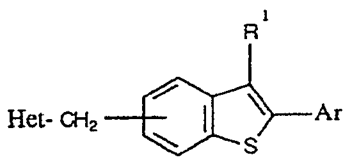

(Ia)

in which $R^3$ in the group Ar represents a nitrile group, with an azide, followed, if necessary by the removal of any protecting group present ;

and when the compound of formula (I) is obtained as a mixture of enantiomers optionally resolving the mixture to obtain the desired enantiomer ;

and/or, if desired, converting the resulting compounds of general formula (I) or a salt thereof into a physiologically acceptable salt, solvate or metabolically labile ester thereof.

**3.** A process as claimed in Claim 1 or 2 for the preparation of a compound of the general formula (I) or a physiologically acceptable salt, solvate or metabolically labile ester thereof

wherein

$R^1$ represents a hydrogen atom or a halogen atom or a group selected from $C_{1-6}$alkyl, $C_{2-6}$alkenyl, —CHO, $-CO_2H$ or $-COR^2$ ;

Ar represents the group

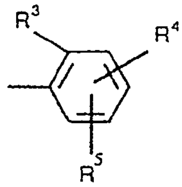 ;

$R^2$ represents a group selected from $C_{1-6}$alkyl, $C_{2-6}$alkenyl, $C_{1-6}$alkoxy or the group $—NR^{10}R^{11}$ ;
$R^3$ represents a group selected from $—CO_2H$, $—NHSO_2CF_3$ or a C-linked tetrazolyl group ;
$R^4$ and $R^5$, which may be the same or different, each independently represent a hydrogen atom or a halogen atom or a $C_{1-6}$alkyl group ;
Het represents an N-linked imidazolyl group substituted at the 2-position by a $C_{1-6}$alkyl, $C_{2-6}$alkenyl, or a $C_{1-6}$alkylthio group, the imidazolyl group optionally being substituted by one or two further substituents selected from a halogen atom or a group selected from cyano, nitro, $C_{1-6}$alkyl, $C_{2-6}$alkenyl, perfluoro$C_{1-3}$alkyl, $—(CH_2)_mR^6$, $—(CH_2)_nCOR^7$ or $—(CH_2)_pNR^8COR^9$ ;
$R^6$ represents a hydroxy or $C_{1-6}$alkoxy group ;
$R^7$ represents a hydrogen atom or a group selected from hydroxy, $C_{1-6}$alkyl, $C_{1-6}$alkoxy, phenyl, phenoxy or the group $—NR^{10}R^{11}$ ;
$R^8$ represents a hydrogen atom or a $C_{1-6}$alkyl group ;
$R^9$ represents a hydrogen atom or a group selected from $C_{1-6}$alkyl, $C_{1-6}$alkoxy, phenyl, phenoxy or the group $—NR^{10}R^{11}$ ;
$R^{10}$ and $R^{11}$ which may be the same or different, each independently represent a hydrogen atom or a $C_{1-4}$alkyl group or $—NR^{10}R^{11}$ forms a saturated heterocyclic ring which has 5 or 6 ring members and may optionally contain in the ring one oxygen atom ;
m represents an integer from 1 to 4 ;
n represents an integer from 0 to 4 ; and
p represents an integer from 1 to 4.

4. A process as claimed in Claim 1 or 2 for the preparation of a compound of the general formula (I) or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, $C_{1-6}$alkoxy, $—CHO$, $—CO_2H$ or $—COR^2$ ;
Ar represents the group

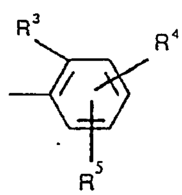 ;

$R^2$ represents a group selected from $C_{1-6}$alkyl, $C_{2-6}$alkenyl, $C_{1-6}$alkoxy or the group $—NR^{10}R^{11}$ ;
$R^3$ represent a group selected from $—CO_2H$, $—NHSO_2CF_3$ or a C-linked tetrazolyl group ;
$R^4$ and $R^5$, which may be the same or different, each independently represent a hydrogen atom or a halogen atom or a $C_{1-6}$alkyl group ;
Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or a group selected from cyano, nitro, $C_{1-6}$alkyl, $C_{2-6}$alkenyl, perfluoro$C_{1-3}$alkyl, $—(CH_2)_mR^6$, $—(CH_2)_nCOR^7$ or $—(CH_2)_pNR^8COR^9$ ;
$R^6$ represents a hydroxy or $C_{1-6}$alkoxy group ;
$R^7$ represents a hydrogen atom or a group selected from hydroxy, $C_{1-6}$alkyl, $C_{1-6}$alkoxy, phenyl, phenoxy

or the group $-NR^{10}R^{11}$ ;
$R^8$ represents a hydrogen atom or a $C_{1-6}$alkyl group ;
$R^9$ represents a hydrogen atom or a group selected from $C_{1-6}$alkyl, $C_{1-6}$alkoxy, phenyl, phenoxy or the group $-NR^{10}R^{11}$ ;
$R^{10}$ and $R^{11}$, which may be the same or different, each independently represent a hydrogen atom or a $C_{1-4}$alkyl group or $-NR^{10}R^{11}$ forms a saturated heterocyclic ring which has 5 or 6 ring members and may optionally contain in the ring one oxygen atom ;
m represents an integer from 1 to 4 ;
n represents an integer from 0 to 4 ; and
p represents an integer from 1 to 4.

5. A process as claimed in any one of Claims 1 to 4 for the preparation of a compound wherein Het is an N-linked imidazolyl group substituted at the 2-position by a hydrogen atom or a $C_{1-5}$alkyl or a $C_{3-5}$alkenyl group, preferably $C_{3-5}$alkyl group, for example an n-butyl group, and the N-linked imidazolyl group is substituted at the 4- and 5- positions by one or two further substituents selected from a halogen atom, preferably a chlorine atom, or a group selected from $C_{1-6}$alkyl, $-(CH_2)_mR^6$ or $-(CH_2)_nCOR^7$, especially $-(CH_2)_nCOR^7$, in which $R^7$ is a hydroxy group and n is 0, 1 or 2.

6. A process as claimed in Claim 5 for the preparation of a compound wherein the N-linked imidazolyl group is substituted by a $-CO_2H$ group.

7. A process as claimed in any one of Claims 1 to 6 for the preparation of a compound wherein the group Het-$CH_2$— is attached at the 5— position on the benzthiophen ring.

8. A process as claimed in any one of Claims 1 and 4 to 7 for the preparation of a compound wherein $R^1$ represents a hydrogen atom or a halogen atom or a group selected from $C_{1-6}$alkyl, $C_{1-6}$alkoxy or fluoro$C_{1-6}$alkyl, preferably a hydrogen atom or a bromine atom.

9. A process as claimed in any one of Claims 1 to 8 for the preparation of a compound wherein $R^3$ represents a C-linked tetrazolyl group.

10. A process as claimed in any one of Claims 1 to 9 for the preparation of a compound wherein $R^4$ and $R^5$ each independently represents a hydrogen atom.

11. A process as claimed in Claim 1 or 2 for the preparation of a compound of the general formula (I) or a physiologically acceptable salt, solvate or metabolically labile ester thereof
wherein
$R^1$ represents a hydrogen atom or a halogen atom ;
Ar represents the group

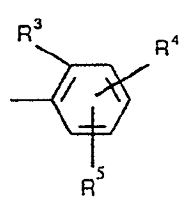 ;

$R^3$ represents a C-linked tetrazolyl group ;
$R^4$ and $R^5$ each independently represent a hydrogen ;
Het represents an N-linked imidazolyl group substituted at the 2-position by a $C_{1-6}$alkyl or group, the imidazolyl group being substituted at the 4- and 5-positions by two further substituents selected from a halogen atom or a, $-(CH_2)_nCOR^7$ group ; $R^7$ represents a hydroxy group ; and
n represents an integer from 0 to 4.

12. A process as claimed in Claim 1 or 2 for the preparation of a compound selected from :
1-[[3-bromo-2-[2-(1H-tetrazol-5-yl)phenyl]-5-benzo[b]thiophenyl]methyl]-2-butyl-4-chloro-1H-imidazo

le-5-carboxylic acid ;
1-[2-[2-(1H-tetrazol-5-yl)phenyl]-5-benzo[b]thiophenyl]methyl]-2-butyl-4-chloro-1H-imidazole-5-carboxylic acid ;
and physiologically acceptable salts, solvates and metabolically labile esters thereof.